# EUROPEAN PATENT APPLICATION

(11) **EP 0 808 846 A2**
(43) Date of publication of application: **26.11.1997**
(21) Application number: 97107975.1
(22) Date of filing: 16.05.1997
(51) Int. Cl.: C07K 14/16, G01N 33/68, A61K 38/16

(54) **Peptides which react with antiboby representing the prognostic marker for HIV disease progression**

(30) Priority: 22.05.1996 GB 9610673; 08.11.1996 GB 9623340
(71) Applicant: Diapharm Limited, St. Peter Port, Guernsey, Channel Islands GY1 4EJ (GB)
(72) Inventor: Veljkovic, Veljko, 11000 Beograd (YU); Metlas, Radmila, 33081 Aviano (PN) (IT)
(74) Representative: Minoja, Fabrizio

(57) **Abstract**

This invention relates to peptide sequences which react with human antibodies whose titre in sera of individuals infected with Human Immunodeficiency Virus type 1 (HIV-1) correlates with Acquired Immune Deficiency Syndrome (AIDS) progression. Hence, these peptides are useful in preparing test kits for the prognostic evaluation of the disease progression, as well as for monitoring the effect of the antiviral therapy on HIV-1 infectivity and modulate immune network. These peptides are also useful in vaccines to prevent the development of AIDS. Monoclonal antibodies against the peptides from this invention may also be used as a therapeutic agent to prevent AIDS progression.

## Description

This invention relates to peptide sequences which react with human antibodies whose titre in the sera of individuals infected with Human Immunodeficiency Virus type 1 (HIV-1) correlates with Acquired Immune Deficiency Syndrome (AIDS) progression. Hence, these peptides are useful in preparing test kits for the prognostic evaluation of the disease progression, as well as for monitoring the effect of antiviral therapy on HIV-1 infected patients. The peptides described in this invention also induce production of antibodies that influence HIV-1 infectivity and modulate the immune network. Hence, these peptides are also useful as vaccines to prevent the development of AIDS. Monoclonal antibodies against the peptides from this invention may also be used as therapeutic agents to prevent AIDS progression.

Investigation of the difference in spectrum of antibodies directed against HIV-1 envelope glycoprotein gp120 in two groups of HIV-infected persons, i.e. those who remained healthy for at least 10 years and those who developed AIDS within 5 years from the onset of infection, showed that the antibody recognising the peptide having the sequence, in the one-letter code, RSANFTDNAKTIIVQLNESVEINCTRP derived from the second conserved region of HIV-1 gp120, is significantly more prevalent in asymptomatic carriers than in AIDS patients [Neurath *et al..,* AIDS Res. Hum. Retrovir. 6, 1183 (1990)]. This result indicates that declining levels or disappearance of this antibody (denoted as NTM.V Ab) may represent a possible factor contributing to the development of AIDS.

The origin of NTM.V Ab remains unknown since the region of HIV-1 gp120 encompassing the peptide pC2 is nonimmunogenic [Bradac and Mathieson, An epitope map to immunity to HIV-1: a roadmap for vaccine development, NIAID, NIH, Bethesda (1991)]. Based on the sequence and spectral homology between HIV-1 gp120-derived peptide RSANFTDNAKTIIVQLNESVEIN (hereinafter "peptide NTM") and human vasoactive intestinal peptide (VIP), it has been suggested that NTM.V Ab probably represents the natural anti-VIP autoantibody [Veljkovic *et al..,* Biochem. Biophys. Res. Commun. 189, 7005 (1992)]. This assumption was confirmed by the strong reactivity of the sera collected from HIV-negative asthma patients, containing high titres of natural anti-VIP antibodies, with the peptide NTM (the main end-point titre for hyperimmune sera was 1/10000) [Veljkovic *et al..,* Biochem. Biophys. Res. Commun. 196, 1019 (1993)].

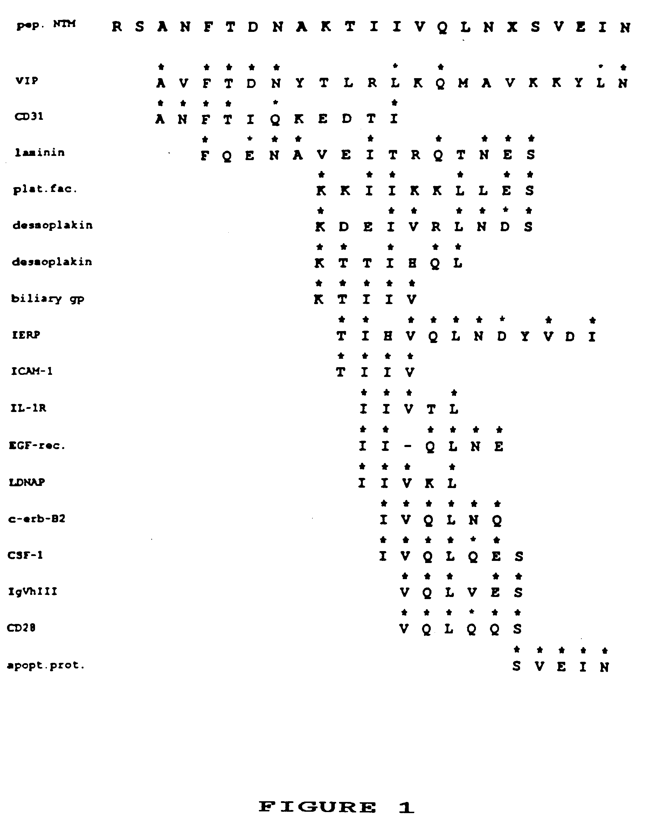

The putative protective role of NTM.V Ab could be ascribed to the following two possibilities:
(1) This antibody blocks the "secondary interaction" between HIV-1 gp120 and CD4 receptor [Veljkovic and Metlas, Cancer Biochem. Biophys. 10, p. 191 (1988); Pollard *et al..,* Proc. Natl. Acad Sci. USA 88, 11320 (1991)], playing an important role in the infection process [Stamatos and Cheng-Mayer, J. Virol. 67, 5635 (1993); Brynmor *et al..,* J. Virol. 67, 7493 (1993)]. This conclusion is in accordance with the fact that jacalin α chain-derived peptide VVVRSLTFKTNKKT, which is highly homologous with the peptide NTM, effectively blocks viral infection *in vivo* without impairing lymphocyte function [Favero *et al..,* Eur. J. Immunol. 23, 179 (1993)].
(2) An unusual set of local homologies between the C-terminus of the second conserved region of HIV-1 gp120 and some human proteins (Figure 1) indicates that this region contains one of the epitopes around which the human immune network is organised [Veljkovic *et al..,* Cancer J. 8, 308 (1995)]. This means that NTM-cross-reactive antibodies could represent important components of the immune network and that their decrease negatively influences the immune network dynamic [Veljkovic and Metlas, Immunol. Today 13, 38 (1992); Metlas and Veljkovic, Vaccine 13, 355 (1995)].

WO 93/17108 discloses analogous peptides of the internal image of a HIV-1 gp120. This reference also relies on the protein informational analysis technique originally developed by the present applicants (Veljkovic V. *et al..,* Phys. Rev. Lett. 29, 105, 1972) and using a set of the HIV-1 gp160, determined the frequency 0.1855 as a main frequency component that correlates with binding of gp120 to CD4 receptor. Based on this frequency, artificial peptides, expressing immunological cross-reactivity with the peptide derived from the C-terminus of HIV-1 gp120, have been designed. The frequency component 0.1855 correlates however with the gp120/gp41 interaction, not with the gp120/CD4 interaction. The above mentioned peptides are, therefore, entirely different from those according to the present invention.

The informational spectrum represents the amplitude spectrum obtained by Fourier transformation of the numerical sequence generated by representation of amino acids in the peptide primary structure with corresponding values of the electron-ion interaction potential (hereinafter EIIP) [Veljkovic *et al..,* IEEE Trans. Biomed. Eng. 32, 337 (1985)]. The values of EIIP for amino acids, calculated according to the General-Model-Pseudopotential [Veljkovic V. and Slavic I., Phys. Rev. Lett. 29, 105 (1972); Veljkovic V. Phys. Lett. 45A, 41 (1973)] and the quasi valence number of amino acids [Veljkovic V. *A Theoretical Approach in Preselection of Carcinogens and Chemical Carcinogenesis,* Gordon & Breach Sci. Pub., New York (1980)] are given in Table 1.

**Table 1**

| Amino acid | EIIP [Ry]* |
|---|---|
| L | O.0000 |
| I | O.0000 |
| N | 0.0036 |
| G | 0.0050 |
| V | 0.0057 |
| E | 0.0058 |
| P | 0.0198 |
| H | 0.242 |
| K | 0.0371 |
| A | 0.373 |
| Y | 0.0516 |
| W | 0.0548 |
| Q | 0.761 |
| M | 0.0823 |
| S | 0.0829 |
| C | 0.0829 |
| T | 0.0941 |
| F | 0.0946 |
| R | 0.0959 |
| D | 0.1263 |

| | |
|---|---|
| * Ry = Rydberg unit | |

HIV-gP120 both of "T-cell line tropic (IIIB, LAV, SF", and RF) and "macrophage-tropic" (HXB2, Ba-1, SF 162 and JR-FL) HIV-1 isolates has been subjected to the above cited "informational spectrum" analysis according to which the IS frequency component F(0.035) is characteristic for the interaction between the "T-cell line tropic" HIV-1 isolates and the fusin molecule.

According to the same analysis, interaction between the CC-CKR-5 receptor and "macrophage-tropic" HIV-1 isolates is characterized by the IS frequency component F(0.0207).

An important task is the identification of region(s) of the HIV-1 gp120 participating in its interaction with the CC-CKR-5 and fusin co-receptors. In order to identify this region, we have scanned primary structures of gp120 molecules from different HIV-1 isolates looking for the region which dominantly contributes to the IS frequency components F(0.035) and F(0.207). This analysis revealed the C-terminus of the second conserved domain (C2) as the most important part of gp120 for its interaction with the co-receptor for both group of HIV-1 isolates, "T-cell line tropic" and "macrophage-tropic" isolates. The consensus peptide VVRSANFTDNAKTIIVQLNESVEINCTRP within C2 domain of gp120 was identified as the most responsible domain for interaction between coreceptor and gp120 for "T-cell line tropic" and "macrophage-tropic" HIV-1 isolates.

The invention provides a peptide derived from the second conserved domain of HIV-1 envelope glycoprotein gp120 presenting in the IS as a dominant the frequency component F1 in the region 0.030-0.040 and/or F2 in the region 0.180-0.220.

More particularly, the peptides of the invention have the general formula I:

X - R₁ - R₂ - D - N - Y (I)

wherein:
X is an amino terminal sequence of arbitrary length and arbitrary amino acid composition;
R₁ is phenylalanine or trypthophan;
R₂ is threonine or serine;
D is aspartic acid;
N is asparagine;
the amino terminal group X is the residue of an amino acid or a peptide sequence having from 2 to 15 amino acids;
the carboxy terminal group Y is the residue of an amino acid or a peptide sequence having from 2 to 15 amino acids, said peptide I immunologically cross-reacting with NTM peptide and with vasoactive intestinal peptide and having in its informational spectrum the dominant frequency component belonging to the frequency interval 0.180 - 0.220, probably 0.207 with the proviso that said peptide is not either the vasoactive intestinal or the NTM peptide.

Advantages of certain of the embodiments of the present invention are as follows:
the peptides are useful as a component of test kit identifying the presence of NTM.V Ab in sera of HIV-1-infected patients - it has been shown that the titre of NTM.V Ab correlates with disease progression so that this antibody is useful as a prognostic marker;
the peptides of the invention are useful as vaccines to give rise to NTM.V Ab to hinder development of AIDS in HIV-1 infected subjects;
the peptides of the invention are also useful as immunogens to elicit monoclonal NTM.V Ab which can be used as a therapeutic agent or prevent development of AIDS or slow its progression; and
antibodies elicited by or reactive with a peptide according to claim 1 or 2 may be useful for preventing AIDS or slowing down its progression.

A strong correlation between reactivity of the peptides of the invention with the sera of HIV-1-infected patients and their stage of disease has been found.

The peptides of the invention may be used as a test reagent in an enzyme-linked immunosorbent assay (ELISA) or an enzyme immunodot assay for the detection of NTM.V Ab. This test may be used for prognosis of disease progression and for monitoring the effect of antiviral and immunotherapy in HIV-1-infected patients. Such test kits may include a porous surface or solid substrate to which the antigenic peptide has been preabsorbed or covalently bound, said surface substrate being preferably in the form of microtitre plates or wells; test sera; various diluents and buffers; labelled conjugates for the detection of specifically bound antibodies and other signal-generating reagents such as enzyme substrates, cofactors and chromogens.

A further aspect of the invention relates to vaccine preparations containing a peptide according to the invention, for protecting against development of AIDS or slowing down its progression in HIV-1 infected patients. Such vaccines contain an effective immunogenic amount of peptide, e.g. 1 µg to 20 mg/kg body weight, optionally conjugated to a protein such as human serum albumin, or expressed on the surface of a suitable viral vector, such as vaccinia virus, in a suitable vehicle, e.g. sterile water, saline or buffered saline. Adjuvants may be employed, such as aluminium hydroxide gel.

The peptides of the invention may be administered by injection, e.g. intradermal, intramuscularly, intraperitoneally, subcutaneously or intravenously. Administration may take place once or at a plurality of times, e.g. at 1 - 4 week intervals.

Antigenic sequences from the crab, for example, as well as proteins from other invertebrates can also be added to the peptides of the invention to promote antigenicity, if so desired.

The peptides according to the invention may be used as immunogens to elicit monoclonal NTM.V Ab using conventional techniques. Such monoclonal antibodies may be used as therapeutic agents, for the prevention of development of AIDS or slowing down its progression.

### Brief description of the drawings

Figure 1 shows the homology between the peptide C-terminus of the second conserved region of HIV-1 gp120 and some human proteins;
Figure 2 shows the comparison of reactivity of the natural peptides NTM and VIP with sera collected from AIDS, ARC and AS patients;
Figure 3 shows the comparison of the reactivity profiles between the peptides NTM and P34 and sera of the HIV-1-infected patients;
Figures 4, 5 and 6 show the relationship between the CD4 count and reactivity of the peptide P34 with sera collected from AIDS, ARC and AS patients, respectively;

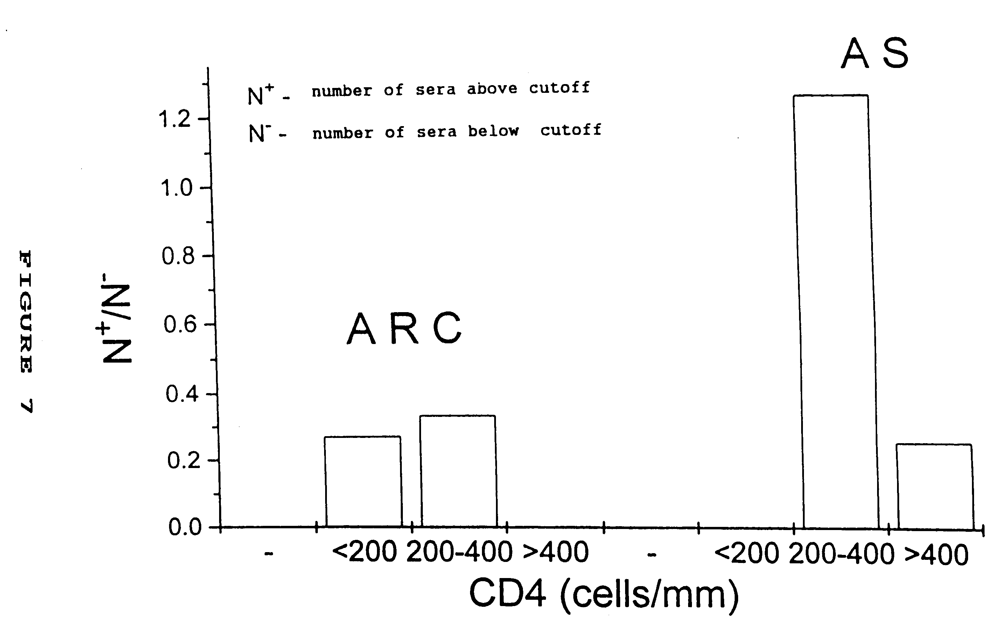

Figure 7 shows the relationship between the CD4 count and ratio between number of sera above and below the cut-off for ARC and AS patients; and
Figures 8, 9, 10 and 11 show the influence of the antiretroviral therapy on reactivity of the peptide P34 with sera collected from ARC and AS patients.
Figure 12 shows the IgG reactivities toward the NTM peptide after heat-inactivation of the sera of 52 healthy individuals (a), and 126 HIV-positive patients (b). The antibody binding of individual patient sera (measured by ELISA assay for heat-inactivated sera dilution 1:100) was presented by single point.
Figure 13 shows the reactivity of non-heat-inactivated sera specimens from Figure 12 with the NTM peptide. (a) Healthy individuals, and (b) HIV-positive patients.
Figure 14 shows effect of temperature denaturation of sera inactivation on IgG reactivities toward the NTM peptide. Heat inactivation of both healthy individuals and HIV-positive patients sera was performed at 40°C and 56°C in duration of 10, 20 and 30 minutes. Reactivity was determined for the NTM peptide coupled to BSA (NTM-BSA _{(EDC)} and the non-coupled NTM peptide.

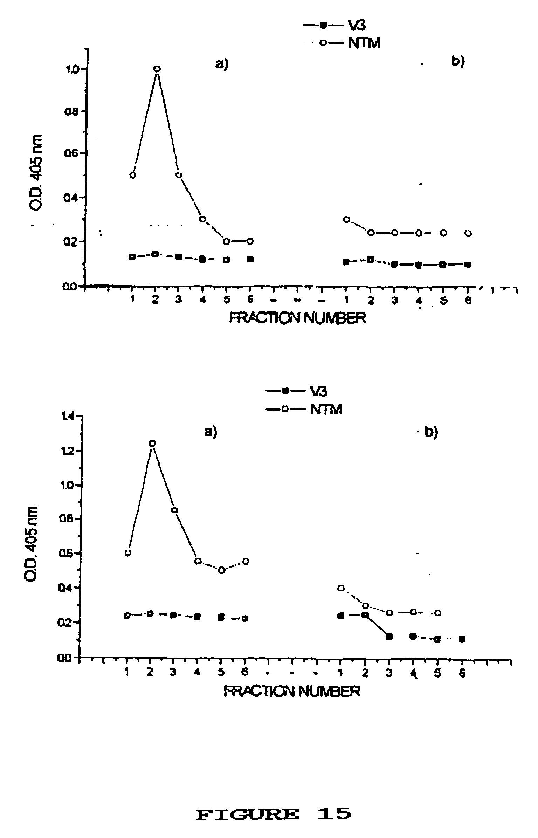

Figure 15 shows binding of antibodies purified by affinity chromatography to the NTM-peptides. Antibodies were purified from sera of healthy individuals (upper panel) and HIV-positive patients (lower panel), both heat-inactivated (a) and non-heat-inactivated sera (b). As control the cross-reactivity profile with the V3 peptide derived from the third hypervariable region of HIV-1 gp120 is added. The V3 peptide was coupled to BSA by glutaraldehyde as a crosslinker [V3-BSA(ga)]. Reactivity was determined in fraction of eluted antibodies from the NTM-BSA coupled to Sepharose-4B column. The NTM peptide was coupled to carrier (BSA) by EDC.

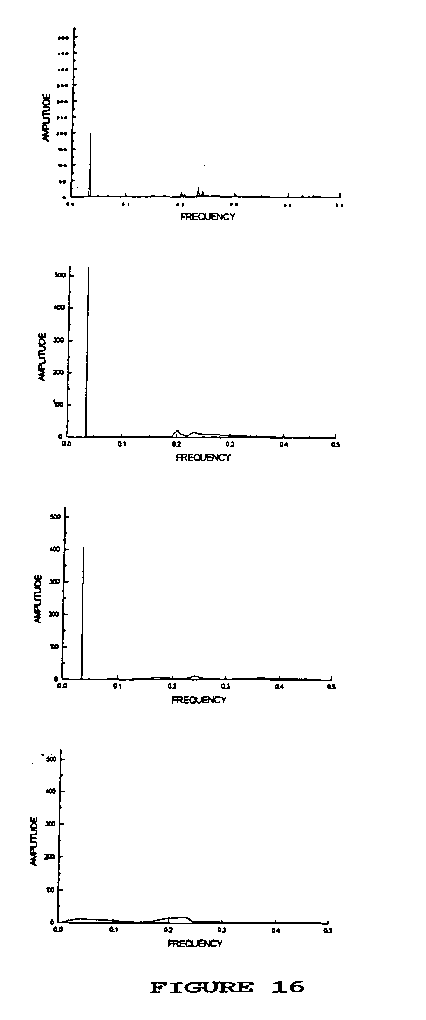

Figure 16 shows (a) the consensus IS of gp120 from "T-cell line tropic" HIV-1 isolates (IIIB, LAV, SF2 and RF), (b) the cross-spectra of the consensus IS of gp 120 from the "T-cell line tropic" HIV-1 isolates and the IS of CD4 receptor, (c) the cross-spectra of the consensus IS of gp120 from "T-cell line tropic" HIV-1 isolates and the fusin receptor, (d) the cross-spectra of the consensus IS of gp120 from "T-cell line tropic" HIV-1 isolates and the CC-CKR-5 receptor.

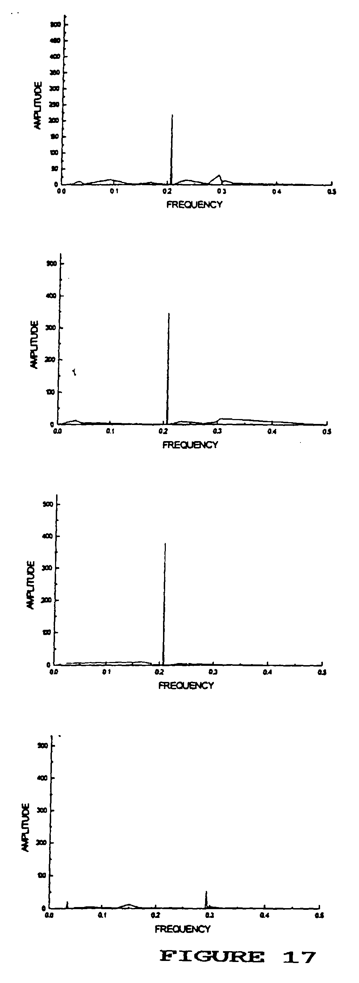

Figure 17 shows (a) the consensus IS of gp120 from the "macrophage-tropic" HIV-1 isolates (HXB2, Ba-1, SF162 and JR-FL), (b) the cross-spectra of the consensus IS of gp120 from the "macrophage-tropic" HIV-1 isolates and the IS of CD4 receptor, (c) the cross-spectra of the consensus IS of gp120 from the "macrophage-tropic" HIV-1 isolates and the CC-CKR-5 receptor, (d) the cross-spectra of the consensus IS of gp120 from the "macrophage-tropic" HIV-1 isolates and the fusin receptor.

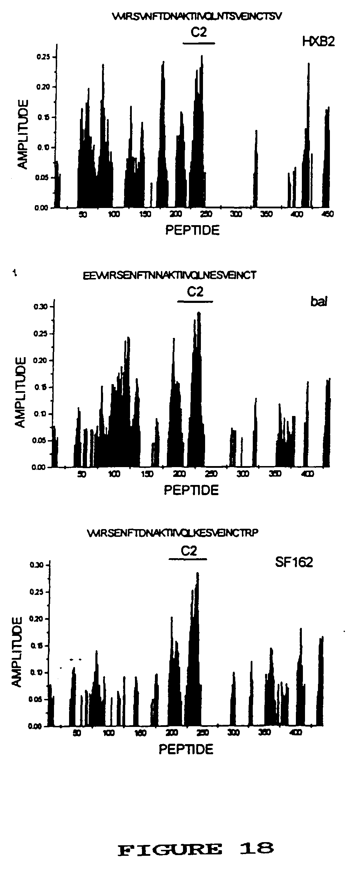

Figure 18 shows the positions of the peptides with S/N ratio > 1 at the IS frequency F(0.207) in the primary structures of gp120 from HXB2, Ba-1 and SF162 HIV-1 isolates.

### EXAMPLES

### Methods

### Study population.

Serum specimens were collected from 194 HIV-1-infected patients divided into three groups:
asymptomatic (AS) patients - 87,
patients with AIDS related complex (ARC) - 85, and
patients who had developed AIDS - 22.

According to the route of infection, patients belonged to the following three groups: intravenous drug users, homosexuals and heterosexuals. Approximately half of the studied patients received antiretroviral therapy (AZT, DDC, DDI, D4T).

As a control, the sera collected from healthy HIV-negative blood donors was used.

### Peptides

The following three artificial peptides of the invention were designed and synthesized according to conventional methods:
- P32 RSAHFTDNAKTPESVEIP: (Sequence Id No. 1)
- P33 RSAHFTDNAKTHPESVEIH: (Sequence Id No. 2)
- P34 HFTDNHPSVEIH: (Sequence Id No. 3)
- P35 VVRSANFTDNAKTIIVQLNESVEINCTRP: (Sequence Id No. 4)

The natural peptides VIP and NTM were also chemically synthesized.

### Enzyme-linked immunosorbent assay.

*Antigen coating* - Peptides were diluted in coating buffer and microtitre plates were coated with 100 µl of peptide 9 solution (1 µg/well). Plates were incubated at 4°C overnight and washed 3 x with phosphate-buffered saline (PBS)/Tween. For each wash, all wells are filled with PBS/Tween prior aspirating and poured on paper towels.
*Blocking* - Remaining binding sites were blocked with 200 µl/well of blocking buffer for 1 h at room temperature and washed 2 x with PBS/Tween.
*Primary antibody reaction* - Sera were diluted in 0.1% Bovine Serum Albumin (BSA) in PBS and 100 µl per well were added. Plates were incubated for 1 h at room temperature, then washed 3 x with PBS/Tween.
*Application of secondary antibody* - Peroxidase labelled goat anti-human IgG antibody, Fc fragment specific diluted 1:5000 in 0.1% BSA in PBS was added at 100 µl per well. After 1 h of incubation at room temperature, the plates were washed 5 x with PBS/Tween, the last one with PBS.
*Substrate* - Colour was developed with ABTS substrates supplemented with H₂0₂. The reaction was stopped after 30 min of incubation by adding 150 µl of 0.1 M citric acid with 0.01% NaN₃, pH 1.8. Absorbance was measured at 450 nm in a microplate reader.
*Reagents*
   Coating buffer: O.1M NaHCO₃ and O.1M Na₂CO₃, pH 9.6; PBS solution: 0.2 g KH₂PO₄, 1.2 g Na₂HPO₄, 8.0 g NaCl and 0.2 g KCl in 1I milliQ water;
   PBS/Tween: 1I PBS solution with 0.5 ml Tween 20 and 0.1 g NaN₃;
   Blocking buffer: 1 % BSA in PBS;
   ABTS substrate; 5 mg ABTS (2,2'-azidobis(3-ethylbenzthiazoline-6-sulfonic acid) in 0.1 ml 0.1 M citric acid 10 pH 4.35 adjusted with NaOH. Prior to use, 4 µl 30% H₂0₂ was added;
   peptide stock solution: 1 mg peptide per 1 ml milliQ water, stored in aliquots at - 20°C.

### Example 1

Experiments have been carried out to compare the reactivity of artificial peptides designed according to the criteria described in this invention with that of VIP and NTM natural peptides with sera of the HIV-1-infected patients.

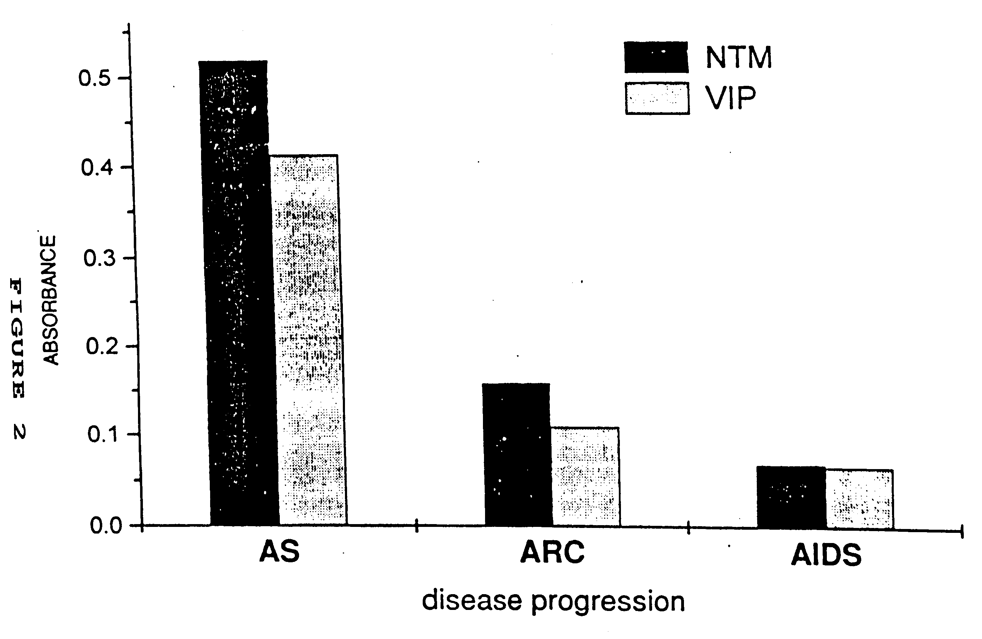

All the artificial peptides P32, P33, P34 and P35 showed a profile of reactivity with HIV-positive sera similar to that of the naturally peptides NTM and VIP. None of the tested peptides has shown a significant reactivity with sera of healthy HIV-negative blood donors. The comparison of the reactivity between VIP, NTM and P34 is reported in Figure 2.

### Example 2

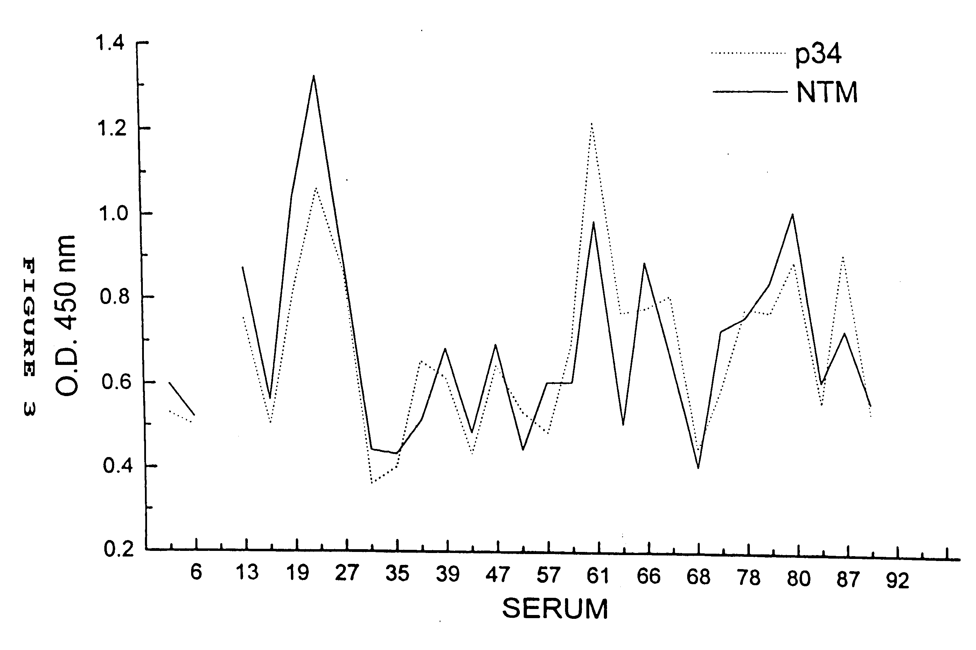

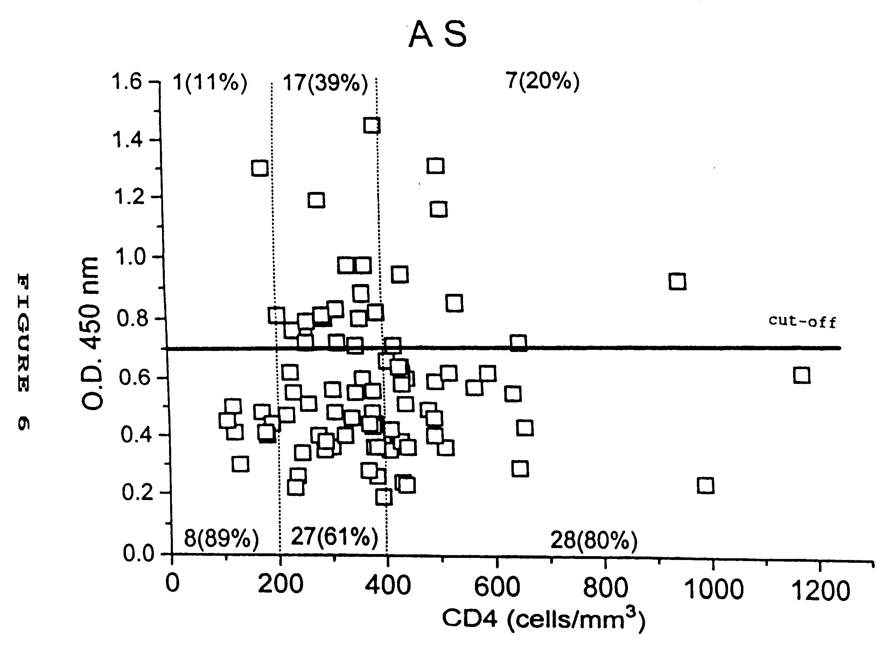

Experiments have been effected to establish the relationship between the reactivity of the peptide P34 with sera of the HIV-1-infected patients and their disease stage. The results of this study are given in Figures 3-6.

The cut-off value of absorbance (O.D.) separating the hyperimmune NTM.V Ab serum from nonhyperimmune serum selected as O.D. cut-off ≥ 0.7, the values were calculated as 3 x O.D.Nₘₑₐₙ of normal serum [Ritchie *et al. ., Methods in Immunology,* Academic Press, New York (1983)]. The O.D.Nₘₑₐₙ obtained as the mean value of 200 normal sera was 0.233.

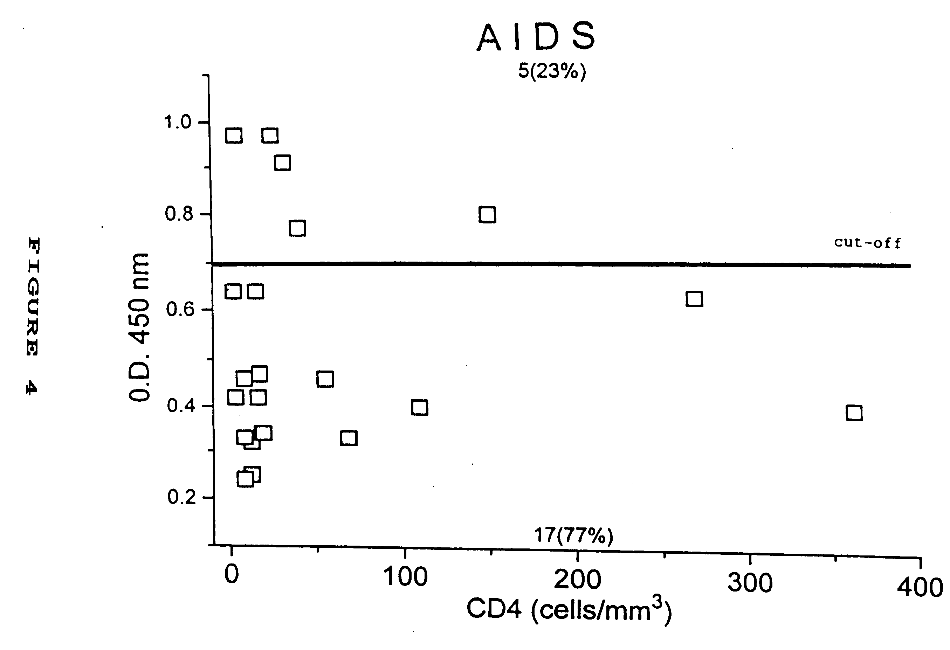

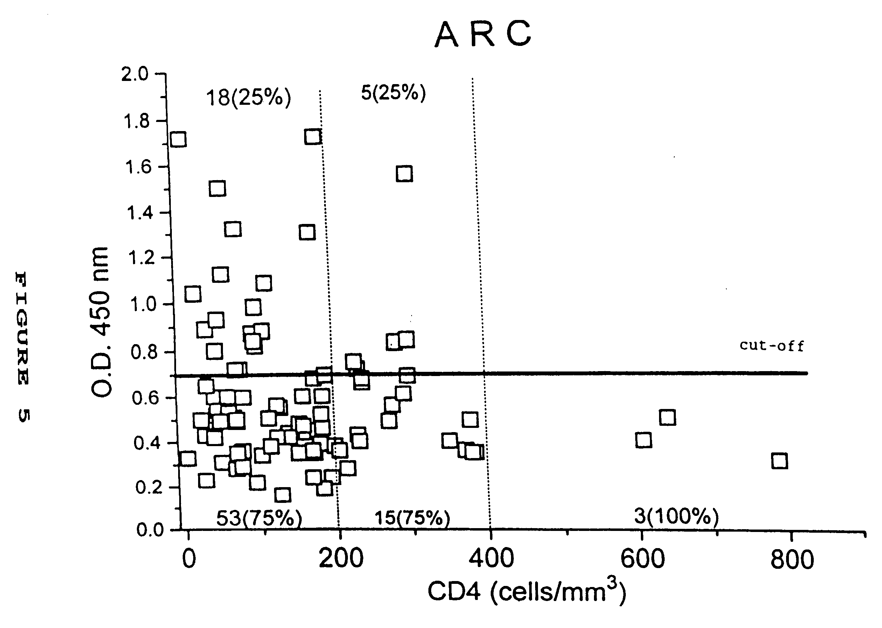

Figures 3 - 6 show that most analysed sera with O.D. ≥ 0.7 correspond to HIV patients with CD4 values between 200 and 400 cells/mm³. It is important to note that in this interval of CD4 count, the ratio of hyperimmune to nonhyperimmune HIV-positive sera is 3.7 times greater for AS than for ARC patients. In the region of CD4 count < 200 the number of patients with the hyperimmune NTM.V Ab sera is remarkably smaller than in patients with nonhyperimmune sera (Figure 3 and 4). The same picture corresponds to the region of CD4 count > 400 (Figure 5).

Based on the above results, the following general pattern for disease evolution could be proposed in terms of NTM.V Ab production in HIV-1-infected patients. At the beginning of infection (CD4 >> 500) the NTM.V Ab titre is low and similar to that of healthy HIV-negative individuals. Near to CD4 count of 500, this antibody increases. In prognostic terms it may be concluded that this increase represents the beginning of disease progression. In the interval of CD4 count 200 - 400, the NTM.V Ab titre remains high for some time and then decreases with further disease progression from AS to ARC and AIDS. Finally, most of ARC and AIDS patients having a low titre of NTM.V Ab have a CD4 count < 200.

The above results show that NTM.V Ab together with CD4 count are useful prognostic markers for monitoring disease progression in HIV-1-infected patients. The peptides according to the invention may be therefore used as antigenic components in a prognostic ELISA test kit, for example.

### Example 3

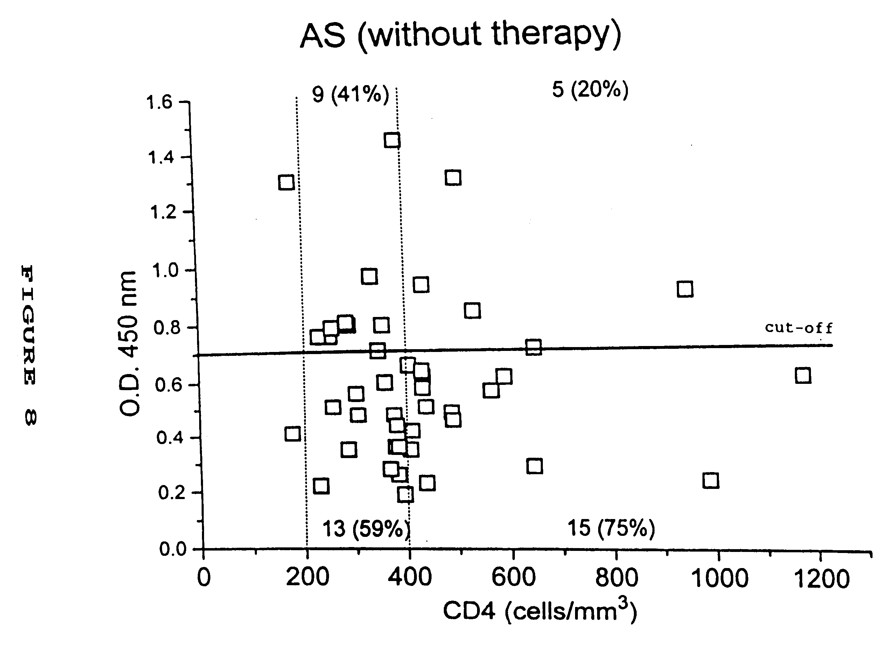

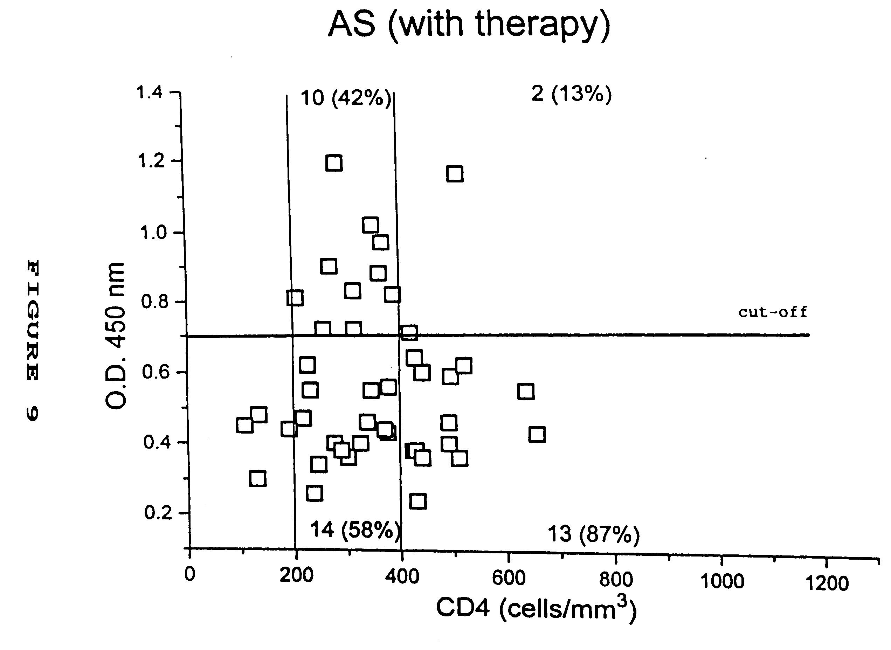

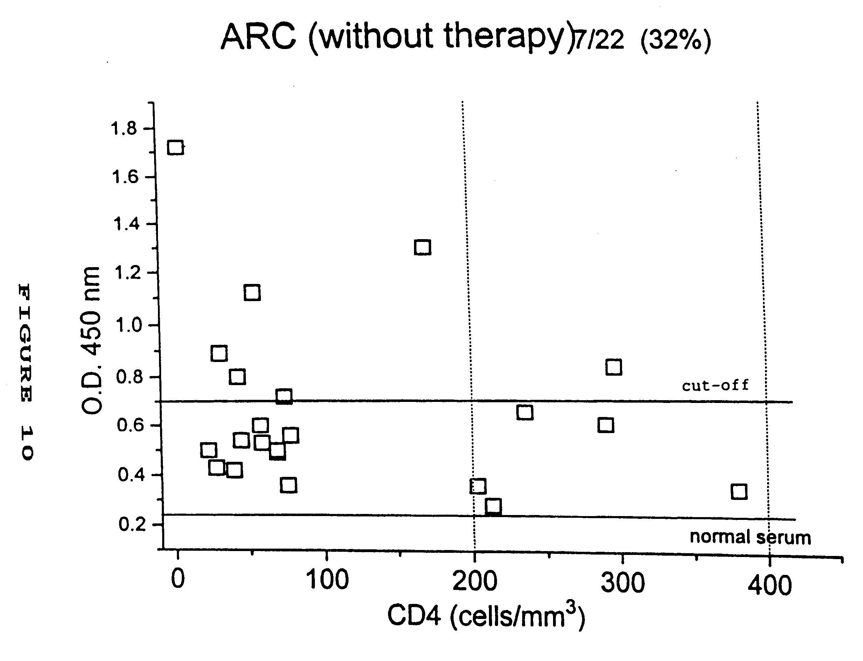

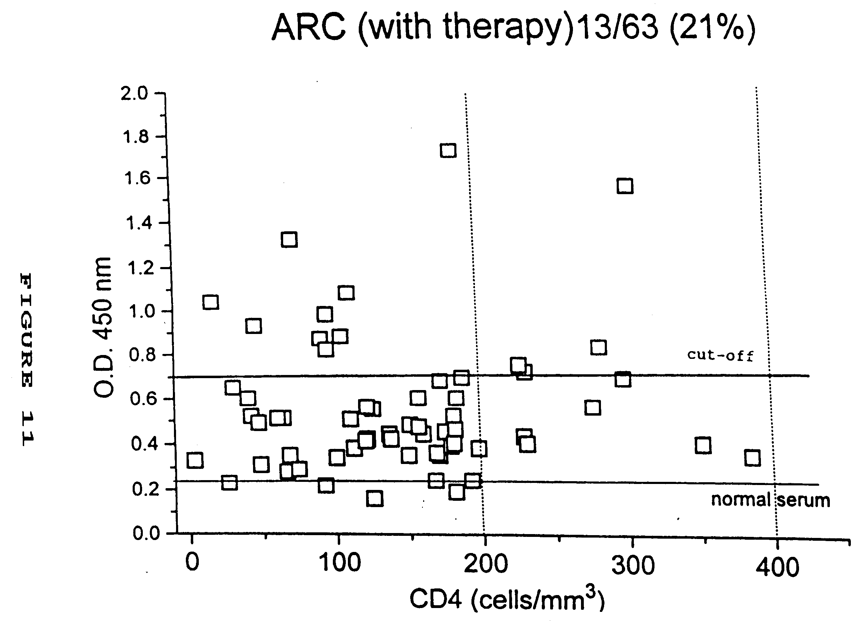

In order to evaluate the effect of the antiretroviral therapy on the NTM.V Ab titre, sera collected from treated and untreated patients were separately analysed. The results reported in Figures 8 - 11 show a positive trend supporting the correlation of therapy in AS patients (CD4 count > 400) with the decrease in NTM.V Ab titre, and with the increase in this antibody titre in ARC patients with CD4 count 200 - 400. On the contrary, in ARC patients with CD4 count < 200, antiretroviral therapy correlates with the decrease in the NTM.V Ab titre. These results are in agreement with the reported effect of the deferred antiretroviral therapy in 13 HIV patients [O'Brien et al.., New Engl. J. Med. 334, 426 (1996)].

### Example 4

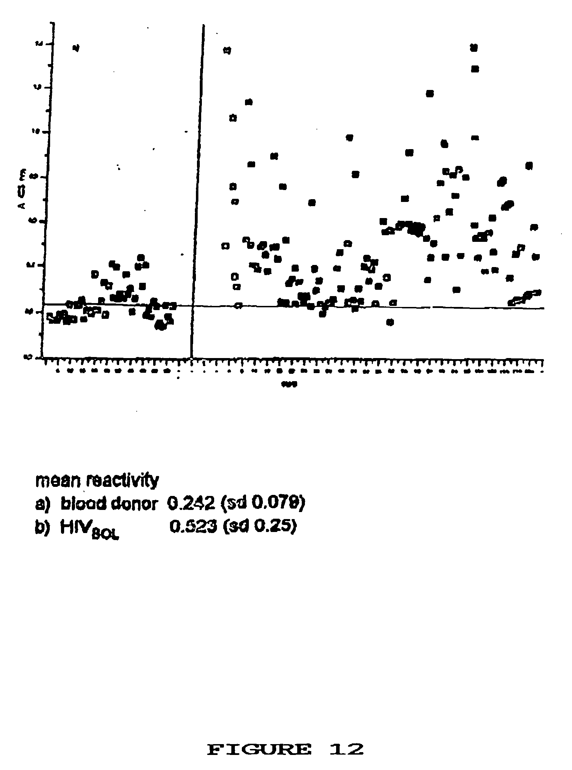

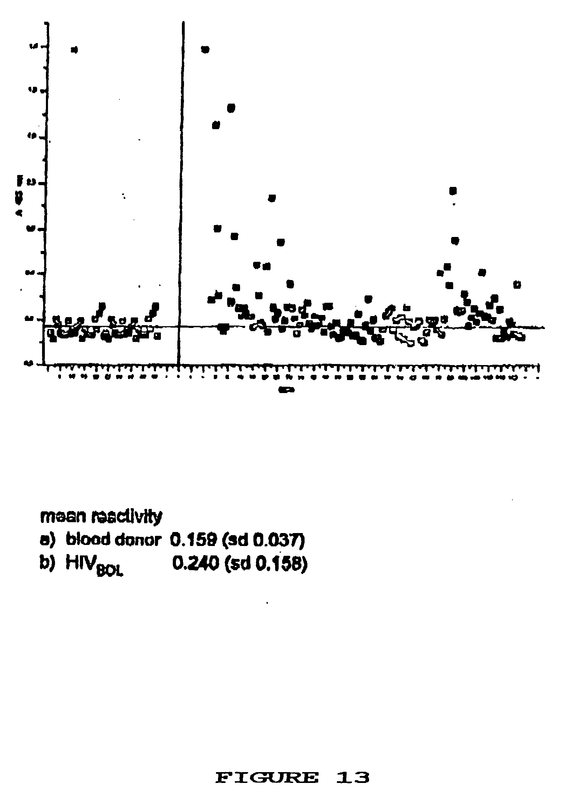

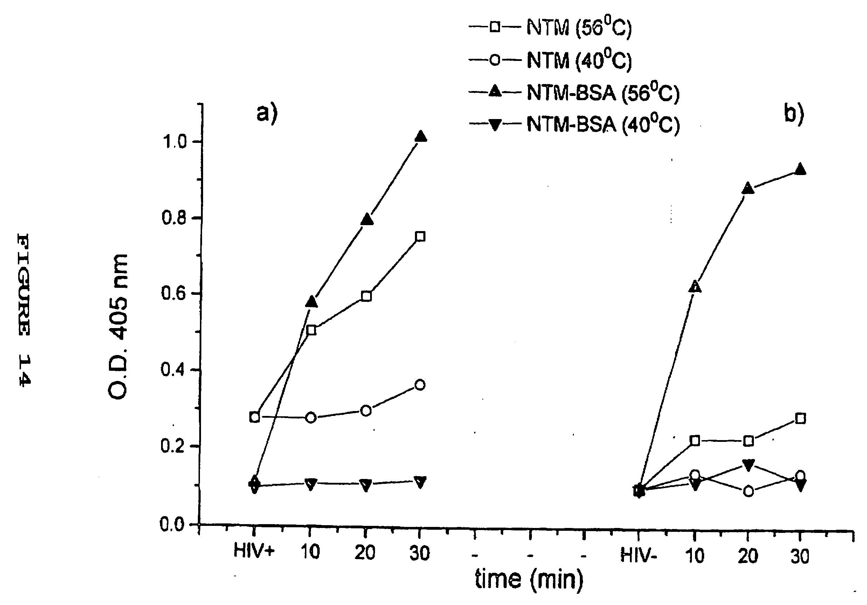

The NTM.V Ab in circulation occurred as heat sensitive immune complexes since very low reactivity was observed with non-heat-inactivated sera of IIIV-positive and healthy individuals (Figure 12) in comparison with reactivity obtained with the same heat-inactivated sera (Figure 13). Reactivity of sera collected from the healthy HIV-negative as well as HIV-positive individuals was examined after heat-inactivation at 40°C and 56°C. The heat treatment of sera was performed in duration of 10, 20 and 30 minutes. Based on the obtained results (Figure 14), it can be concluded that release of the NTM.V Ab from immune complexes is temperature and time dependent. In these experiments IgG reactivity toward two forms of the NTM peptide were monitored, i.e. uncoupled and BSA-coupled forms. The reactivity patterns of both NTM forms were similar.

### Example 5

Experiments performed with affinity purified antibodies on the NTM-carrier-coupled-Sepharose 4B column confirmed a heat-sensitive nature of the NTM.V Ab described in the Example 4. According to these results (Figure 15) the affinity purification of the NTM.V Ab can be only obtained from the heat-inactivated serum collected from the healthy HIV-negative, as well as HIV-positive individuals.

### Example 6

In order to determine which of the characteristic frequencies from IS of the gp120 from the "T-cell line tropic" HIV-1 isolates are responsible for their interaction with the CD4 receptor and the fusin co-receptor, the ISM analysis of these proteins has been performed.

These analysis revealed that the consensus IS (CIS) of gp120 from the IIIB, LAV and RF HIV-1 isolates (Fig. 1a), cross-spectra between this CIS and IS of the CD4 receptor (Fig.1b), as well as cross-spectra between this CIS and IS of the fusin molecule (Fig. 1c) contain only one characteristic peak corresponding to the frequency component F(0.035). Contrary, cross-spectra between CIS of gp120 from analyzed "T-cell tropic" HlV-1 isolates and IS of the CC-CKR-5 molecule, representing the co-receptor of the "macrophage-tropic" isolates, do not contain any characteristic peak above the noise (Fig. 1d).

### Example 7

In order to determine which of the characteristic frequencies from IS of the gp120 from the "macrophage-tropic" HIV-1 isolates are responsible for their interaction with the CD4 receptor and the CC-CKR-5 co-receptor, the ISM analysis of these proteins has been performed. These analysis revealed that the consensus IS (CIS) of gp120 from the HXB2, Ba-1, SF 162 and JR-FL HIV-1 isolates (Fig. 2a), cross-spectra between this CIS and IS of the CD4 receptor (Fig. 2b), as well as cross-spectra between this CIS and IS of the CC-CKR-5 molecule (Fig. 2c) contain only one characteristic peak corresponding to the frequency component F(0.207). Contrary, the cross-spectra between CIS of gp120 from analyzed "macrophage-tropic" HIV-1 isolates and IS of the fusin molecule, representing co-receptor of the "T-cell tropic" isolates, does not contain any characteristic peak above the noise (Fig. 2d).

According to these analysis it can be concluded that the IS frequency component F(0.207) is characteristic for the interaction between gp120 from "macrophage-tropic" HIV-1 isolates and the CD4 receptor are the CC-CKR-5 co-receptor.

### Example 8

In order to identify the domain of gp120 from the "macrophage-tropic" HIV-1 isolates with the maximal amplitude at the IS frequency F(0.207), their primary structures were scanned with peptides of different lengths (from 20 to 30 a.a.). Results of this analysis for HXB2, Ba-1 and SF160 isolates are given in Fig. 3. These analysis revealed that the consensus peptide **VVRSANFTDNAKTIIVQLNESVEINCTRP** from the C-terminus of the C2 domain of gp120 from the "macrophage-tropic" HIV-1 isolates represents the region with the highest amplitude corresponding to the frequency component 10 F(0.207). It means that this region of the "macrophage-tropic" HIV-1 isolates is most important for their interaction with the CC-CK1R-5 co-receptor.

SEQUENCE LISTING

(1) GENERAL INFORMATION:

(i) APPLICANT:
(A) NAME: diapharm
(B) STREET: 123
(C) CITY: 333
(D) STATE: 3333
(E) COUNTRY: 33333
(F) POSTAL CODE (ZIP): 122222

(ii) TITLE OF INVENTION: polypeptides

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version
#1.30 (EPO)

(2) INFORMATION FOR SEQ ID NO: 1:

(i) SEQUENCE CHARACTERISTICS:
(A) LENGTH: 18 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 1:

Arg Ser Ala His Phe Thr Asp Asn Ala Lys Thr Pro Glu Ser
1               5                   10
Val Glu Ile Pro
15

(2) INFORMATION FOR SEQ ID NO: 2:

(i) SEQUENCE CHARACTERISTICS:
(A) LENGTH: 19 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Arg Ser Ala His Phe Thr Asp Asn Ala Lys Thr His Pro Glu
1               5                   10
Ser Val Glu Ile His
15

(2) INFORMATION FOR SEQ ID NO: 3:

(i) SEQUENCE CHARACTERISTICS:
(A) LENGTH: 12 amino acids
(B) TYPE: amino acid
(C) STRANDEDNESS:

(D) TOPOLOGY: linear

(ii) MOLECULE TYPE: peptide

(xi) SEQUENCE DESCRIPTION: SEQ ID NO: 3:

His Phe Thr Asp Asn His Pro Ser Val Glu Ile His
1               5                   10

(2) INFORMATION FOR SEQ ID NO: 4:

(i) SEQUENCE CHARACTERISTICS:
(A) LENGTH: 29 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 4:

Val Val Arg Ser Ala Asn Phe Thr Asp Asn Ala Lys Thr Ile
1               5                   10
Ile Val Gln Leu Asn Glu Ser Val Glu Ile Asn Cys Thr Arg
15                  20                  25
Pro

## Claims

1. A peptide derived from the second conserved domain of HIV-1 envelope glycoprotein gp120 presenting in the IS as a dominant the frequency component F1 in the region 0.030-0.040 and/or F2 in the region 0.180-0.220.

2. A peptide having the general formula I:
X - R₁ - R₂ - D - N - Y (I)
wherein:
X is an amino terminal sequence of arbitrary length and arbitrary amino acid composition;
R₁ is phenylalanine or trypthophan;
R₂ is threonine or serine;
D is aspartic acid;
N is asparagine;
the amino terminal group X is the residue of an amino acid or a peptide sequence having from 2 to 15 amino acids;
the carboxy terminal group Y is the residue of an amino acid or a peptide sequence having from 2 to 15 amino acids, said peptide immunologically cross-reacting with NTM peptide and with vasoactive intestinal peptide and having in its informational spectrum the dominant frequency component belonging to the frequency interval 0.180 - O.220 with the proviso that said peptide is neither the vasoactive intestinal nor the NTM peptide.

3. A peptide according to claim 1 or 2 having the sequence selected from:
RSAHFTDNASTPESVEIP
RSAHFTDNAKTHPESVEIH
HFTDNHPSVEIH
VVRSANFTDNAKTIIVQLNESVEINCTRP.

4. A test kit for the detection of NTM.V antibodies recognising the peptide NTM (RSANFTDNAKTIIVQLNESVEIN) derived from the second conserved region of HIV-1 and human VIP, containing an antigenic peptide according to Claim 2 or 3, bound to a porous surface or a solid substrate.

5. A test kit according to Claim 4, wherein the antigen peptide is bound to wells of a microtitre plate.

6. A pharmaceutical composition containing as the active ingredient a peptide as claimed in Claim 1, 2 or 3 together with a pharmaceutical carrier therefor.

7. A composition of matter comprising a peptide according to Claim 1 or 2 conjugated to a protein.

8. A composition according to Claim 7 wherein the protein is human serum albumin.

9. A composition of matter comprising a peptide according to Claim 1, 2 or 3 expressed on the surface of a viral vector.

10. A composition according to claim 9 wherein the viral vector is vaccinia virus.

11. A pharmaceutical composition containing as the active ingredient an affinity purified antibody obtained from the human HIV-positive and/or HIV-negative sera heated above 40°C, using the peptide-carrier-coupled column, where peptide is defined according to claim 1 or 2.

12. Use of a peptide according to Claim 1, 2 or 3, for the manufacture of a medicament for preventing AIDS or slowing down its progression.

13. Use of the antibodies elicited by or reactive with a peptide according to Claim 1, 2 or 3, for preventing AIDS or slowing down its progression.

14. A peptide as defined in claim 1, 2 or 3 for use in anti-AIDS therapy.

15. An antibody capable of recognising a peptide as defined in claim 1, 2 or 3, for use in anti-AIDS therapy.
